Europäisches Patentamt

European Patent Office

Office européen des brevets

(11) Numéro de publication: **0 362 768**
**A1**

## (12) DEMANDE DE BREVET EUROPEEN

(21) Numéro de dépôt: **89118235.4**

(22) Date de dépôt: **02.10.89**

(51) Int. Cl.5: **A61B 3/00**

(30) Priorité: **06.10.88 CH 3742/88**
**10.10.88 FR 8813400**

(43) Date de publication de la demande:
**11.04.90 Bulletin 90/15**

(84) Etats contractants désignés:
**AT BE DE GB IT LU NL SE**

(71) Demandeur: **LASAG AG**
**Mittlere Strasse 52**
**CH-3600 Thun(CH)**

(72) Inventeur: **Nouri, Taoufik**
**Oberstadelstrasse 14**
**CH-3653 Oberhofen(CH)**
Inventeur: **Bätscher, Paul**
**Löwenmattweg 11**
**CH-3110 Münsingen(CH)**

(74) Mandataire: **Barbeaux, Bernard et al**
**ICB Ingénieurs Conseils en Brevets SA**
**Passage Max. Meuron 6**
**CH-2001 Neuchâtel(CH)**

(54) **Dispositif pour caler la tête d'un patient notamment pendant un examen ou un traitement ophtalmologique.**

(57) Un dispositif pour maintenir la tête d'un patient dans une position prédéterminée, notamment lors d'un examen ou d'un traitement ophtalmologique, ledit dispositif comprend une base (10), une traverse horizontale (1) qui porte des moyens (2', 2") pour caler le front du patient, des moyens de support (5, 6) de cette traverse, des moyens (8) pour caler le menton du patient et des moyens de support (7) de ces moyens de calage du menton.

Grâce à la présence d'un montant latéral unique (5) dont la partie supérieure porte la traverse (1), le dispositif laisse une grande liberté de manoeuvre au médecin, tout en permettant de caler la tête du patient de manière efficace.

FIG. 2

## DISPOSITIF POUR CALER LA TETE D'UN PATIENT NOTAMMENT PENDANT UN EXAMEN OU UN TRAITE-MENT OPHTALMOLOGIQUE

La présente invention concerne le domaine du matériel médical.

Plus précisément, la présente invention a pour objet un nouveau dispositif pour caler la tête d'un patient en position verticale, notamment lors d'un examen ou d'un traitement ophtalmologique et lorsque le patient est assis.

Des dispositifs connus de ce genre sont décrits dans les brevets US 4 128 317 et 4 139 280. Ces dispositifs connus comprennent deux colonnes verticales, réunies par une traverse horizontale supérieure et une traverse horizontale inférieure. La traverse supérieure est destinée à caler le front du patient et la traverse inférieure destinée à caler son menton. Les deux colonnes sont fixées sur une base qui peut être un plateau de travail qui porte également un ou plusieurs appareils d'examen ou de traitement, ou un socle fixé sur ce plateau. De plus, ces dispositifs comprennent des moyens pour modifier la position de la traverse inférieure, le long des colonnes verticales. Le brevet US 4 128 317 décrit en outre des moyens pour modifier également la position de la traverse supérieure, le long de ces deux colonnes.

De tels dispositifs permettent de caler la tête du patient de manière satisfaisante par rapport au plateau de travail mais, ils ne laissent que peu de liberté de manoeuvre au médecin. En effet, la traverse supérieure est relativement large, plaquée sur le front du patient et arrive au-dessous des sourcils de celui-ci. Quant au deux colonnes verticales, elles encadrent son visage de chaque côté, au niveau de ses joues et, en avant de ses oreilles. De tels dispositifs rendent donc malaisées certaines opérations délicates, telles que la pose de lentille de contact, préalablement à de la chirurgie laser par exemple.

Le but de l'invention est de fournir un dispositif qui laisse un maximum de liberté de manoeuvre au médecin, tout en calant la tête du patient de manière efficace.

Ce but est atteint grâce au fait que dans le dispositif selon l'invention qui comprend, comme les dispositifs connus dont on vient de parler, une base, une traverse horizontale qui s'étend de part et d'autre d'un plan vertical avec lequel le plan de symétrie de la tête du patient doit être sensiblement confondu et qui porte des premiers moyens de calage pour caler le front du patient, des moyens de support de cette traverse, des seconds moyens de calage pour caler le menton du patient et des moyens de support de ces seconds moyens de calage, les moyens de support de la traverse horizontale comprennent un montant latéral unique dont la partie supérieure porte la traverse, par l'une de ses extrémités, et dont la partie inférieure est liée rigidement à la base du dispositif.

Grâce à cette caractéristique essentielle, le dispositif de l'invention permet une liberté de manoeuvre maximale pour le médecin du côté opposé à celui du montant unique, tout en assurant un maintien efficace de la tête du patient, dans une position correcte.

D'autres caractéristiques et avantages de la présente invention apparaîtront à la lecture de la description détaillée qui suit, donnée uniquement à titre d'exemple non limitatif et faite en se référant aux dessins annexés, sur lesquels :

- la figure 1 montre schématiquement, de face et en cours d'utilisation un mode de réalisation préféré du dispositif selon l'invention;

- les figures 2, 3 et 4 sont des vues schématiques respectivement de face, de profil et de dessus de ce même mode de réalisation;

- la figure 5 est une vue schmématique en coupe partielle, selon le plan V-V de la figure 3, qui met en évidence les moyens pour faire varier la distance entre les moyens pour caler le front et ceux pour caler le menton du patient dont le dispositif des figures 2 à 4 est muni;

- la figure 6 est une vue schématique d'arrière de la partie supérieure d'une variante possible du mode d'exécution des figures 1 à 5;

- la figure 7 est vue schématique et partielle de dessus d'une autre variante possible de ce mode d'exécution; et

- la figure 8 est une vue schématique en coupe partielle de la partie inférieure de cette même variante.

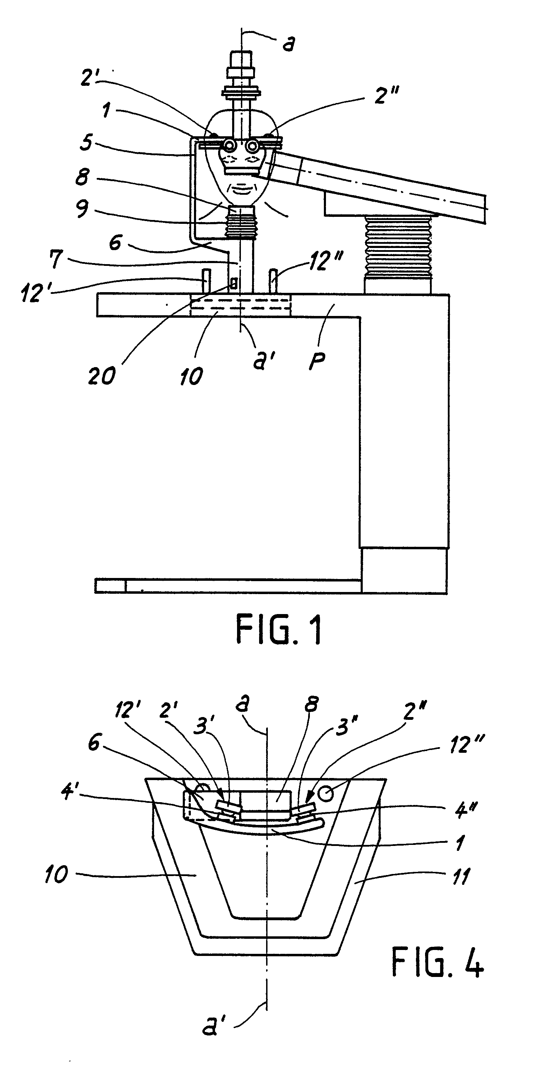

Le dispositif selon l'invention qui est représenté sur les figures 1 à 4 est prévu pour être monté de façon amovible sur un plateau de travail P que l'on peut voir sur la figure 1.

La traverse horizontale de ce dispositif est constituée par une barre rigide 1 qui est légèrement incurvée vers l'arrière de celui-ci pour pouvoir suivre à peu près le contour du front d'un patient.

Dans ce mode d'exécution que l'on a choisi comme exemple cette barre 1 porte, comme moyens pour caler le front du patient, des éléments d'appui fixes 2' et 2" qui sont disposés symétriquement par rapport à un plan vertical a-a' lié au dispositif et avec lequel le plan de symétrie de la tête du patient doit être sensiblement confondu lorsque celle-ci est correctement calée.

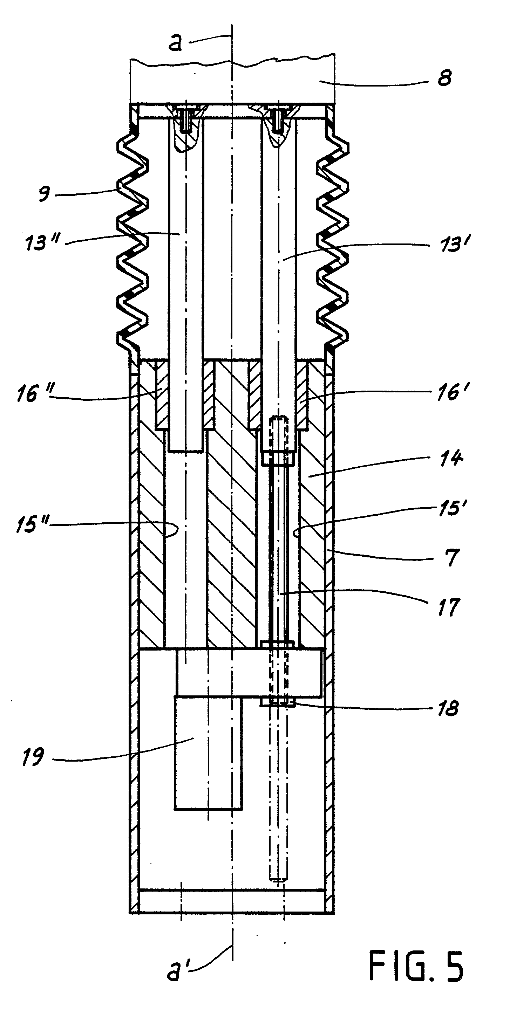

Comme on peut le voir sur la figure 4, chacun de ces éléments 2' et 2" comprend une partie en forme de disque ou de coupelle évasée 3', respectivement 3″, contre laquelle l'un des côtés du front du patient doit venir s'appuyer, et un pied 4′, respectivement 4″, par lequel cette partie est fixée à la barre 1.

Conformément à l'invention cette barre 1 est portée par l'une de ses extrémités par la partie supérieure d'un montant vertical et latéral unique 5 tandis que son autre extrémité est libre.

Tels qu'ils sont représentés sur le dessins, ces deux éléments 1 et 5 sont réunis bout à bout mais il est évident que le montant pourrait monter un peu plus haut que la barre et/ou que la barre pourrait se prolonger un peu au-delà du montant.

L'essentiel est que le montant soit suffisamment écarté du plan a-a′ pour ne pas risquer de gêner le médecin lors d'un examen ou d'un traitement et qu'il présente une résistance à la flexion élevée dans la direction parallèle à ce plan a-a′, c'est-à-dire celle dans laquelle le patient exerce une force sur la barre 1 lorsqu'il appuie son front contre les éléments 2′ et 2″. Pour avoir cette rigidité le montant 5 peut être constitué par exemple par une barre à section rectangulaire disposée de façon que la longueur de cette section soit parallèle au plan a-a′.

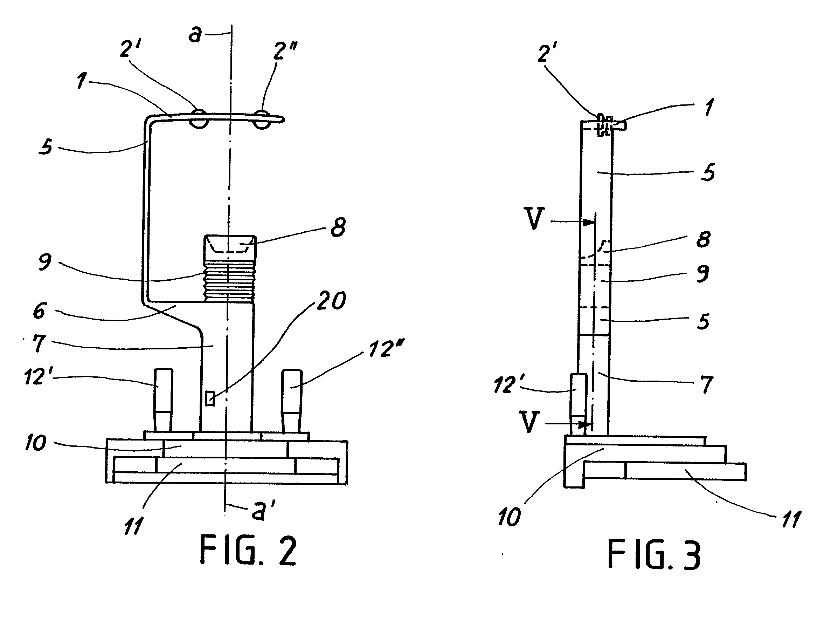

Comme le montrent les figures 1 et 2, le montant 5 est porté par l'une des extrémités d'un bras horizontal 6 dont l'autre extrémité est solidaire d'une colonne verticale creuse 7, qui présente un plan de symétrie confondu avec le plan a-a′ et qui est fixée, par exemple par des vis, sur un socle 10.

Cette colonne 7, qui a par exemple une section rectangulaire ou carrée, constitue l'un des éléments des moyens de support de ceux qui servent à caler le menton du patient et qui sont constitués ici par une mentonnière 8.

Les autres éléments de ces moyens de support sont ceux d'un système télescopique qui porte la mentonnière 8 et qui permet de la soulever ou de l'abaisser pour régler la distance entre elle et la barre 1.

Ce système qui est logé principalement dans la colonne 7 et dont la partie qui émerge de celle-ci est entourée par un soufflet 9 qui sera décrit par la suite.

D'autre part, dans ce mode d'exécution, le socle 10 du dispositif est conçu pour pouvoir venir s'emboîter facilement dans une encoche qui est située en bordure du plateau de travail P dont on a parlé, et qui a une forme qui correspond à la sienne.

Plus précisément, ce socle est constitué par une pièce de forme sensiblement trapézoïdale (voir figure 4) qui comporte, sauf du côté de sa grande base, un bord aminci 11 destiné à venir s'engager dans une rainure que le plateau présente sur sa tranche, autour de son encoche également trapézoïdale. De plus, le socle 10 et l'encoche du plateau sont prévus pour que, lorsque le socle est en place, sa grande base soit alignée avec le bord du plateau.

Ainsi, le dispositif selon l'invention peut être fixé sur le plateau de travail de façon à la fois amovible et très stable. Par ailleurs, lorsque le dispositif est retiré de l'encoche, celle-ci constitue un espace libre dans lequel peut s'inscrire la tête d'un patient allongé sous le plateau de travail, sur les yeux duquel un médecin peut intervenir, ou dans lequel peuvent être placés des moyens pour positionner la tête de ce patient.

Enfin, comme on peut le voir sur l'ensemble des figures 1 à 4, le dispositif comporte également deux poignées 12′ et 12″ qui sont fixées sur le socle 10, de part et d'autre de la colonne 7. Grâce à ces poignées le patient qui est assis devant le plateau de travail peut plus facilement avancer la tête pour la caler sur le dispositif et la maintenir ensuite immobile pendant un examen ou une intervention.

La figure 5 montre schématiquement comment est réalisé le système télescopique qui porte la mentonnière 8 et qui permet de régler la hauteur de celle-ci par rapport au socle 10.

Ce système comprend deux barres cylindriques verticales 13′ et 13″ qui sont situées symétriquement par rapport au plan a-a′ et aux extrémités supérieures desquelles est fixée la mentonnière 8, par exemple par des vis, ainsi que des moyens de guidage et des moyens d'entraînement pour permettre de déplacer ces barres axialement dans les deux sens.

Les moyens de guidage comprennent un bloc métallique allongé 14 de même section que la section interne de la colonne 7, qui est logé entièrement dans la partie supérieure de celle-ci et qui en est solidaire.

Ce bloc présente dans le sens de sa longueur deux alésages parallèles 15′ et 15″ dans lesquels les barres 13′ et 13″ sont engagées et peuvent pénétrer plus au moins profondément en coulissant dans deux douilles 16′ et 16″ chassées dans la partie supérieure de ces alésages.

Les moyens d'entraînement comprennent, eux, une vis sans fin 17 qui est fixée par un bout à l'extrémité inférieure de l'une, 13′, des barres verticales et située dans le prolongement de celle-ci, un écrou 18 dans lequel passe la vis 17 et qui est maintenu en place axialement par des moyens non représentés, et un moteur électrique réversible 19 qui permet de faire tourner l'écrou dans les deux sens, par l'intermédiaire de moyens de transmission également non représentés, et par conséquent de faire monter ou descendre la vis 17.

D'après la description qui vient d'en être faite il est facile de prévoir comment le dispositif des figures 1 à 5 peut être utilisé.

Après s'être assis devant le plateau de travail P le patient prend les poignées 12′ et 12″ en main, et avance sa tête pour venir appuyer son front contre les éléments 2′ et 2″. Si la mentonnière 8 n'est pas alors à la bonne hauteur pour que le patient puisse venir en même temps y caler son menton le médecin utilise le bouton 20 situé en bas de la colonne 7, que l'on peut voir sur les figures 1 et 2, pour faire fonctionner le moteur 19 dans un sens ou dans l'autre et faire monter ou descendre la mentonnière jusqu'à ce qu'elle se trouve en position correcte. Ensuite, lorsque le patient a bien calé son front sur les éléments d'appui et son menton sur la mentonnière, le médecin peut procéder à l'examen ou à l'intervention qu'il souhaite faire.

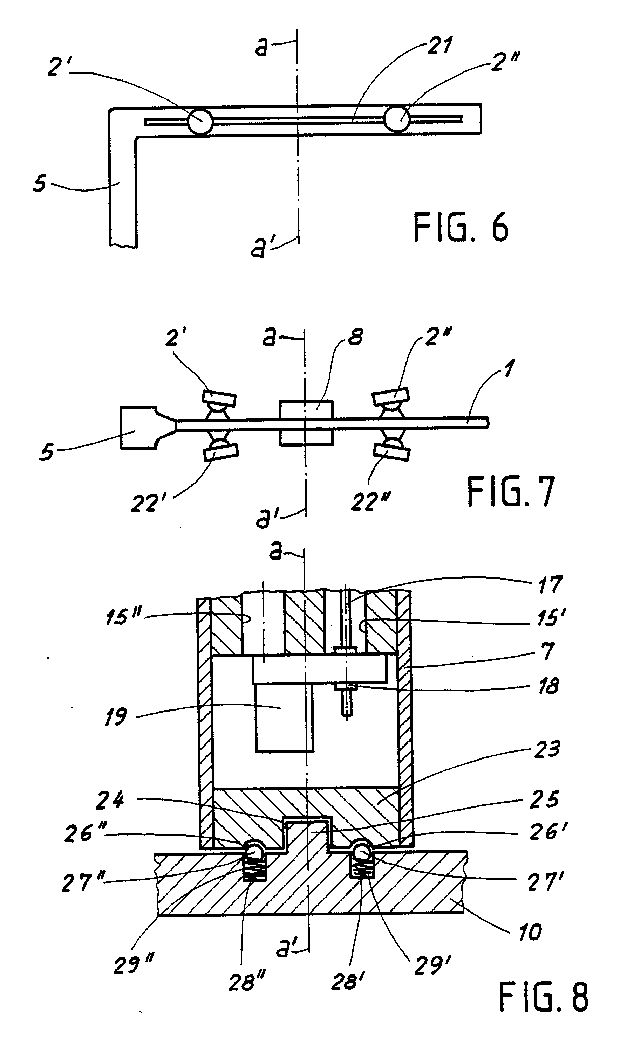

La seule différence entre le mode d'exécution des figures 1 à 5 et la variante qui est représentée à la fois très schématiquement et très partiellement sur la figure 6 c'est que, dans cette dernière, la traverse horizontale 1 porte un rail longitudinal 21 le long duquel les éléments d'appui du front 2′ et 2″ peuvent être déplacés. Grâce à cela il est possible d'utiliser le même dispositif pour des patients qui ont des fronts de largeurs très différentes.

Dans la variante illustrée par les figures 7 et 8, le dispositif est conçu pour permettre de placer le montant latéral 5 qui porte la traverse horizontale 1 à gauche ou à droite du visage du patient, selon que le médecin désire avoir une liberté de manoeuvre d'un côté ou de l'autre de celui-ci. En d'autres termes, le dispositif est prévu pour pouvoir être utilisé dans deux positions symétriques par rapport au plan vertical a-a′.

Pour cela, comme le montre la figure 7, la traverse horizontale 1 n'est plus incurvée mais droite et elle ne porte plus seulement deux éléments d'appui 2′ et 2″ pour le front mais quatre, les deux éléments d'appui supplémentaires 22′ et 22″ étant placés évidemment du côté opposé à celui des éléments 2′ et 2″.

D'autre part, le dispositif est muni d'un système comme celui de la figure 8, qui permet de faire tourner la colonne 7 autour d'un axe vertical contenu dans le plan a-a′ et de la stabiliser dans deux positions à 180° l'une de l'autre.

Dans ce cas, la colonne 7 comprend un fond 23 qui présente à l'extérieur, un trou central cylindrique 24 dans lequel est engagé un bossage de même forme et de même diamètre 25, que comporte le socle 10, et deux autres trous en forme de calottes sphériques 26′ et 26″ qui sont symétriques par rapport à l'axe du trou 24 et dans lesquels peuvent pénétrer deux billes 27′ et 27″ qui émergent plus ou moins de deux trous cylindriques correspondants 28′ et 28″ du socle 10, sous la poussée de deux ressorts 29′ et 29″ logés dans ces trous 28′ et 28″.

Naturellement, la profondeur des trous 26′ et 26″ et la force que les ressorts 29′ et 29″ exercent sur les billes 27′ et 27″ doivent être suffisantes pour que la colonne 7 ne risque pas de tourner sous la seule action des forces que le patient peut exercer sur le dispositif mais uniquement lorsque le médecin le désire.

Enfin, toujours dans cette même variante, le dispositif peut comporter soit une mentonnière 8 fixe, que l'on peut utiliser de deux côtés, soit une mentonnière amovible à laquelle on peut faire faire un demi-tour autour de l'axe de rotation de la colonne.

Une telle variante est particulièrement avantageuse car elle permet au médecin d'avoir une liberté de manoeuvre maximale des deux côtés du visage du patient, alternativement.

Bien entendu, on pourrait aussi imaginer une autre variante dans laquelle ce ne serait pas la colonne 7 qui tournerait mais seulement une bague qui serait située au-dessus de cette colonne et qui porterait le bras horizontal, le montant et la traverse. Dans ce cas, la mentonnière et le système qui la porte et qui permet d'en régler la hauteur pourraient être les mêmes que ceux du mode d'exécution des figures 1 à 5.

Cela dit, il est clair que l'invention n'est pas limitée à ce mode d'exécution ni aux variantes que l'on vient de décrire ou d'envisager.

Par exemple, plutôt que d'être droit et vertical le montant latéral pourrait être oblique et conformé pour passer derrière l'oreille du patient et, indépendamment de cela, il pourrait être fixé sur le socle du dispositif au lieu d'être porté par les moyens de support de la mentonnière.

On pourrait aussi concevoir un mode d'exécution dans lequel le montant serait fixé de cette façon, c'est-à-dire sur le socle, et dans lequel les moyens de support de la mentonnière seraient constitués par un bras horizontal rigide qui serait porté par l'une de ses extrémités par le montant, de façon que sa hauteur puisse être réglée manuellement.

Dans d'autres modes d'exécution on pourrait avoir à la fois une mentonnière et une traverse horizontale ou seulement une traverse horizontale de hauteur réglable.

Par ailleurs, dans certains cas, la base du dispositif pourrait ne pas être un socle fixé de façon amovible ou non sur un plateau de travail mais le plateau de travail lui-même.

**Revendications**

1. Dispositif pour caler la tête d'un patient en position verticale, notamment lors d'un examen ou d'un traitement ophtalmologique, comprenant une base (10), une traverse horizontale (1) qui s'étend de part et d'autre d'un plan vertical (a-a') avec lequel le plan de symétrie de la tête du patient doit être sensiblement confondu et qui porte des premiers moyens de calage (2',2") pour caler le front du patient, des moyens de support (5, 6) de cette traverse, des seconds moyens de calage (8) pour caler le menton du patient et des moyens de support (7,13',13",14,15',15",16',16", 17,18,19) de ces seconds moyens de calage, caractérisé par le fait que les moyens de support de la traverse horizontale comprennent un montant latéral unique (5) dont la partie supérieure porte ladite traverse par l'une de ses extrémités et dont la partie inférieure est liée rigidement à ladite base.

2. Dispositif selon la revendication 1, caractérisé par le fait que les moyens de support (7,13',13",14,15',15",16',16",17,18,19) des seconds moyens de calage (8) sont conçus pour permettre de faire varier la distance entre ces seconds moyens de calage et les premiers moyens de calage (2',2").

3. Dispositif selon la revendication 2, caractérisé par le fait que les moyens de support des seconds moyens de calage (8) comprennent une colonne verticale creuse (7), solidaire de ladite base (10) et ayant pour plan de symétrie ledit plan vertical (a-a'), une barre verticale (13') montée coulissante à l'intérieur de ladite colonne et à l'extrémité supérieure de laquelle sont fixés lesdits seconds moyens de calage et des moyens d'entraînement (17,18,19) pour déplacer verticalement ladite barre.

4. Dispositif selon la revendication 3, caractérisé par le fait que les moyens d'entraînement de la barre (13') comprennent une vis sans fin (17) solidaire de ladite barre et qui prolonge celle-ci à l'intérieur de ladite colonne (7) et un moteur réversible (19) couplé mécaniquement à cette vis.

5. Dispositif selon la revendication 3 ou 4, caractérisé par le fait que les moyens de support des seconds moyens de calage (8) comprennent en plus une seconde barre (13") parallèle à la première (13'), qui peut également coulisser à l'intérieur de ladite colonne (7) et à l'extrémité supérieure de laquelle lesdits seconds moyens de calage (8) sont également fixés, et par le fait que les deux barres sont sensiblement symétriques par rapport audit plan vertical (a-a').

6. Dispositif selon l'une quelconque des revendications 3 à 5, caractérisé par le fait que la partie inférieure dudit montant latéral (5) est solidaire de l'une des extrémités d'un bras sensiblement horizontal (6) dont l'autre extrémité est solidaire de ladite colonne (7).

7. Dispositif selon l'une quelconque des revendications précédentes, caractérisé par le fait que les premiers moyens de calage sont constitués par deux éléments d'appui (2',2") pour le front, disposés de part et d'autre dudit plan vertical (a-a').

8. Dispositif selon la revendication 7, caractérisé par le fait que ladite traverse (1) est munie d'un rail longitudinal (21) le long duquel lesdits éléments d'appui (2',2") peuvent être déplacés.

9. Dispositif selon l'une quelconque des revendications précédentes, caractérisé par le fait que ladite base est constituée par un socle (10) qui permet de fixer le dispositif de façon amovible sur un plateau de travail (P).

10. Dispositif selon la revendication 9, caractérisé par le fait que ledit socle (10) est conçu pour pouvoir s'emboîter dans une encoche de forme correspondante qui est prévue en bordure du plateau de travail (P) et qui, en l'absence du dispositif, constitue un espace libre dans lequel peut s'inscrire la tête d'un patient allongé sous ledit plateau de travail ou dans lequel peuvent être placés des moyens pour positionner celle-ci.

11. Dispositif selon la revendication 10, caractérisé par le fait que ledit socle (10) a sensiblement la forme d'un trapèze dont la grande base est alignée avec le bord du plateau de travail (P) lorsque ce socle est emboîté dans ladite encoche.

12. Dispositif selon l'une quelconque des revendications précédentes, caractérisé par le fait qu'il comprend en plus des moyens (22',22",24-29") qui permettent de l'utiliser dans deux positions symétriques par rapport audit plan vertical (a-a') afin que ledit montant latéral (5) puisse se trouver d'un côté ou de l'autre du visage du patient.

FIG. 1

FIG. 4

EP 0 362 768 A1

FIG. 2

FIG. 3

FIG. 5

EP 0 362 768 A1

FIG. 6

FIG. 7

FIG. 8

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | FR-A-2 322 576 (CARL ZEISS) <br> * Page 1, ligne 35 - page 2, ligne 25; page 2, ligne 35 - page 3, ligne 22; figures 1-3 * & US-A-4 139 280 (Cat. D) | 1,2,9 | A 61 B 3/00 |
| A | | 4,6 | |
| Y | EP-A-0 038 525 (CARL ZEISS) <br> * Résumé; page 3, lignes 4-19; figure 1 * | 1,2,9 | |
| A | | 7 | |
| A | FR-A-2 381 506 (A.I. IGNATIADES) <br> * Page 2, lignes 1-18; figures 1,2 * | 1,4,9 | |
| A | FR-A-1 149 085 (MAISON LUER) <br> * Page 1, colonne de droite, lignes 8-35; figures 1,2 * | 1,2,4,7,9 | |
| A | DE-U-8 512 249 (CARL ZEISS) <br> * Page 2, lignes 13-28; figure 1 * | 3,4,9 | |
| D,A | US-A-4 128 317 (M. LECOVER) <br> * Résumé; colonne 2, lignes 21-60; colonne 3, lignes 18-24,33-41; figures 1-4 * | 1,2,12 | **DOMAINES TECHNIQUES RECHERCHES (Int. Cl.5)** <br><br> A 61 B |

Le présent rapport a été établi pour toutes les revendications

| Lieu de la recherche | Date d'achèvement de la recherche | Examinateur |
|---|---|---|
| LA HAYE | 22-12-1989 | RIEB K.D. |